# EUROPÄISCHE PATENTSCHRIFT

(11) **EP 4 106 833 B1**
(45) Veröffentlichungstag und Bekanntmachung des Hinweises auf die Patenterteilung: **22.04.2026**
(21) Anmeldenummer: 21703159.0
(22) Anmeldetag: 25.01.2021
(51) Int. Cl.: A61M 1/16

(54) **FLUIDFLUSSSTEUERUNG EINER BLUTBEHANDLUNGSVORRICHTUNG**
CONTROLLING FLUID FLOWING IN A BLOOD TREATMENT DEVICE
CONTRÔLE DE LA CIRCULATION DE FLUID DANS UN DISPOSITIF DE TRAITEMENT DE SANG

(30) Priorität: 17.02.2020 DE 102020104101
(43) Veröffentlichungstag der Anmeldung: 28.12.2022
(73) Patentinhaber: Fresenius Medical Care Deutschland GmbH, 61352 Bad Homburg (DE)
(72) Erfinder: IRRGANG, Tobias, 97633 Aubstadt (DE)
(74) Vertreter: Rau, Jenspeter
(86) Internationale Anmeldenummer: PCT/EP2021/051610
(87) Internationale Veröffentlichungsnummer: WO 2021/164989

(56) Entgegenhaltungen:
- WO-A1-01/95956
- DE-A1- 102011 106 111
- DE-A1- 102017 001 770
- US-A1- 2009 124 963
- US-A1- 2011 315 611
- US-A1- 2013 336 814

## Beschreibung

### Technisches Gebiet

Die vorliegende Erfindung betrifft eine Blutbehandlungsvorrichtung sowie ein Verfahren zum Steuern eines Fluidflusses in einer Blutbehandlungsvorrichtung.

### Hintergrund

Unter dem Begriff der Blutbehandlungsvorrichtung ist unter anderem eine Dialysemaschine zu verstehen. Dialysemaschinen finden häufig in Dialysezentren zur Behandlung einer chronischen Nierenerkrankung Anwendung. Neben hohen Sicherheitsanforderungen spielt bei der Anwendung von Dialysemaschinen in Dialysezentren der zeitliche Aspekt eine besondere Rolle.

Vor allem ist die Einhaltung genauer Zeitpläne bei dieser Anwendung von Blutbehandlungsvorrichtungen, insbesondere bei Dialysebehandlungen, aus nachfolgend genannten Gründen essentiell. Zum einen sind die bei Dialysebehandlungen zum Einsatz kommenden Dialysemaschinen in einem Behandlungszentrum regelmäßig derart den einzelnen Behandlungen verschiedener Dialysepatienten zugeordnet, dass bei einer Behandlungsdauer von 4 bis 5 Stunden an einer Dialysemaschine drei Personen am Tag behandelt werden können.

Auch die zwischen den Behandlungen zur Vorbereitung der zu behandelnden Person, dem Patienten, sowie andere Schritte, wie z.B. die zur Desinfizierung der Dialysemaschine benötigte Dauer, ist einer ständigen Zeitoptimierung unterworfen. Verzögert sich eine Behandlung, müssen die nachfolgenden Behandlungen und damit die Zeitpläne der Patienten aber auch die Zeitpläne des behandelnden Personals, dem Pflegepersonal oder Nephrologen, geändert werden. Folgen einer solchen Abänderung können zu Überstunden und Stress des behandelnden Personals als auch zu Kosten für das jeweilige Behandlungszentrum beispielsweise aufgrund einer ungünstigen Auslastung der Dialysemaschinen führen.

Aufgrund dieser engen Taktung sollte nicht nur die Zeit zwischen den Behandlungen möglichst kurzgehalten werden, auch Unterbrechungen während einer Behandlung müssen möglichst gering gehalten werden.

Verschieben sich aufgrund häufiger Unterbrechungen einer Behandlung die nachfolgenden Behandlungen, ergeben sich hieraus nicht nur Nachteile für das Behandlungszentrum und Personal, Verschiebungen einer Behandlung haben auch nachteilige Auswirkungen auf den Patienten.

Verschieben sich Behandlungen etwa auf den nachfolgenden Tag oder, aufgrund von Feiertagen sowie Wochenenden, sogar um mehrere Tage, kann dies zu gesundheitsgefährdenden Folgen führen. Starke Überwässerung sowie die ansteigende Vergiftung durch den Aufbau von harnpflichtigen Substanzen wirken sich kritisch auf die Gesundheit des Patienten aus.

Zudem wirken sich lange Behandlungen negativ auf das Wohlbefinden des Patienten aus. Zielsetzung ist es, einem Patienten trotz regelmäßiger Behandlungen ein möglichst normales Alltagsleben zu ermöglichen. Die Behandlung soll damit nicht mehr Zeit als nötig in Anspruch nehmen.

Neben der oben aufgeführten zeitkritischen Behandlungssituation ist es vorteilhaft, die einwandfreie Funktionsweise der Blutbehandlungsvorrichtung ständig zu überwachen, um die Sicherheit des Patienten gewährleisten zu können.

Um die Sicherheit während einer Dialysebehandlung zu gewährleisten, sind verschiedene Sicherheitssysteme bekannt. Zur Überprüfung der einwandfreien Funktionsweise kann bei der Durchführung einiger Sicherheitstests die Behandlung unterbrochen werden. Als Sicherheitssystem ist beispielsweise ein Druckhaltetest aus der DE 4239937 C2 bekannt.

Zu Beginn dieses Druckhaltetests wird eine vorgegebene Menge an Flüssigkeit in den Dialysierflüssigkeitskreislauf gefördert. Ein Abflussventil ist während des Druckhaltetests geschlossen, so dass ein vorbestimmter Druck aufgebaut werden kann. Der Dialysierflüssigkeitskreislauf ist während dieses Tests in einem erweiterten Bypass, so dass die Flüssigkeit nicht in den Dialysator gelangen kann.

Der Verlauf des Flüssigkeitsdrucks in dem Dialysierflüssigkeitskreislauf kann aus dem an einer oder mehreren Stellen im Dialysierflüssigkeitskreislauf gemessenen Druckwertes bestimmt werden. Beispielsweise wird der Druckhaltetest für eine Dauer von 8 Sekunden und periodisch während der Behandlung durchgeführt. Gelangt nun zur Durchführung des Druckhaltetests eine nicht physiologische (unphysiologische) Flüssigkeit in den Dialysierflüssigkeitskreislauf, muss diese vor der Fortsetzung der Behandlung aus dem Dialysierflüssigkeitskreislauf entfernt werden, um zu vermeiden, dass unphysiologische Flüssigkeit zum Patienten gelangt.

Zur Erkennung einer unphysiologischen Flüssigkeit ist in dem Dialysierflüssigkeitskreislauf beispielsweise ein Leitfähigkeitssensor zur Überwachung der Zusammensetzung der Flüssigkeit vorgesehen. Wird unphysiologische Flüssigkeit mittels des Leitfähigkeitssensors erfasst, hat diese Flüssigkeit damit bereits den Dialysierflüssigkeitskreislauf erreicht. Der Leitfähigkeitssensor kann dazu den gemessenen Wert mit einem Sollwert oder Sollwertbereich vergleichen und die Blutbehandlungsvorrichtung bzw. die Steuerung derselben kann bei einer Abweichung eine unphysiologische Flüssigkeit erkennen.

Um die unphysiologische Flüssigkeit aus dem Dialysierflüssigkeitskreislauf zu entfernen, sollte nach einer positiven Detektion einer unphysiologischen Flüssigkeit ein zeitintensives Spülen durchgeführt werden, wodurch die Unterbrechung der Behandlung verlängert wird. Daraus können sich die oben angeführten negativen Effekte ergeben.

Im Stand der Technik gibt es Bestrebungen, die Unterbrechung der Behandlung aufgrund des Druckhaltetests gering zu halten. Beispielsweise offenbart die EP 1327457 B1 ein Verfahren zur Detektion von Leckagen im Flüssigkeitssystem, das die Überwachung ohne Unterbrechung der Blutbehandlung aufgrund des Durchführens eines Druckhaltetests erlaubt. Damit wird ein Druckhaltetest erst dann durchgeführt, wenn eine hohe Wahrscheinlichkeit einer Leckage vorliegt. Wird eine hohe Wahrscheinlichkeit für eine Leckage detektiert, wird damit auch bei diesem Verfahren die Behandlung unterbrochen und ein Druckhaltetest durchgeführt.

Der vorliegenden Anmeldung liegt damit die Aufgabe zu Grunde, die Unterbrechung der Blutbehandlung, insbesondere aufgrund der Durchführung eines Druckhaltetests möglichst gering zu halten und gleichzeitig die Sicherheit des Patienten zu gewährleisten.

### Zusammenfassung der Erfindung

Die der Erfindung zugrunde liegende Aufgabe wird durch die Blutbehandlungsvorrichtung gemäß Anspruch 1 sowie das Verfahren zum Steuern eines Fluidflusses in einer Blutbehandlungsvorrichtung nach Anspruch 13 gelöst. Vorteilhafte Weiterentwicklungen und Ausführungsformen sind Gegenstand der abhängigen Ansprüche.

Erfindungsgemäß ist eine Blutbehandlungsvorrichtung vorgesehen, welche ein Fluidleitungssystem zum Leiten eines Fluidflusses mit einem Leitungsabschnitt und zumindest einer ersten Konzentratzufuhrleitung zum Zuführen einer ersten Konzentratlösung aufweist. Der Leitungsabschnitt ist dabei als ein geschlossenes Fluidsystem ausbildbar. Ferner weist die Blutbehandlungsvorrichtung eine Fluidpumpe zum Fördern eines Fluids in dem Fluidleitungssystem sowie ein Bestimmungsmittel zum Erfassen eines Zustands des Fluidleitungssystems und eine Steuereinheit zum Steuern des Fluidflusses auf. Die Blutbehandlungsvorrichtung ist dadurch gekennzeichnet, dass die Steuereinheit derart konfiguriert ist, dass der Leitungsabschnitt nur dann ein geschlossenes Fluidsystem ausbildet, wenn der vom Bestimmungsmittel erfasste Zustand eine vorgegebene Bedingung erfüllt.

Ein geschlossenes Fluidsystem ist erfindungsgemäß ein Fluidsystem, in welches weder ein Zufluss noch ein Abfluss eines Fluids stattfindet. Dabei kommt das Fluid nach einer gewissen Zeit in dem geschlossenen Fluidsystem zum Stillstand, das heißt, es findet kein Fluidfluss mehr statt, so dass der in dem geschlossenen Fluidsystem vorherrschende dynamische Druck gegen null geht und der Gesamtdruck annähernd dem statischen Druck entspricht.

Das Fluidleitungssystem weist dabei einen Leitungsabschnitt auf, welcher als ein geschlossenen Fluidsystem ausgebildet werden kann. Mit anderen Worten kann ein Teilabschnitt des Fluidleitungssystem derart abgetrennt werden, dass das Fluid in diesem Teilabschnitt gehalten werden kann.

Das Fluidleitungssystem weist neben dem Leitungsabschnitt eine Konzentratzufuhrleitung auf. Diese Konzentratzufuhrleitung ist mit dem Leitungsabschnitt derart direkt oder indirekt verbunden, dass ein über die Konzentratzufuhrleitung gefördertes Fluid, etwa eine Konzentratlösung, in den Leitungsabschnitt gelangen kann.

Die Steuereinheit steuert den Fluidfluss durch das gezielte Ansteuern von zumindest einer in der Blutbehandlungsvorrichtung angeordneten Fluidpumpe und/oder Absperrelementen. Diese durch die Steuereinheit ansteuerbaren Elemente können dabei auch außerhalb der Blutbehandlungsvorrichtung angeordnet sein.

Das Bestimmungsmittel erfasst einen Zustand des Fluidleitungssystems. Insbesondere erfasst das Bestimmungsmittel einen Zustand des in dem Fluidleitungssystems vorliegenden Fluids. Dabei kann das Bestimmungsmittel ein Fluidsensor sein. Alternativ oder zugleich kann das Bestimmungsmittel ein Sensor zum Erfassen einer Position eines Verbindungsmittels sein, das heißt, das Bestimmungsmittel kann mehrere physische Sensoren aufweisen, beispielsweise einen Leitfähigkeitssensor und/oder einen Leitwertsensor und/oder einen Magnetsensor und/oder einen Kontaktsensor. Mit anderen Worten kann das Bestimmungsmittel mehrere Elemente aufweisen. Dabei kann das Bestimmungsmittel auch nur zwei Fluidsensoren aufweisen, die an unterschiedlichen Positionen in dem Fluidleitungssystem angeordnet sind.

Nur dann, wenn der durch das Bestimmungsmittel erfasste Zustand des Fluidleitungssystems eine vorgegebene Bedingung erfüllt, wird der Leitungsabschnitt geschlossen, das heißt, es wird ein geschlossenes Fluidsystem ausgebildet.

Die vorgegebene Bedingung des Zustands des Fluidleitungssystems kann dabei einen Wertbereich angeben, welcher durch eine Ober- und/oder Untergrenze definiert ist. Die vorgegebene Bedingung kann aber auch nur ein Vorhandensein erfassen. Beispielsweise kann die vorgegebene Bedingung definiert sein, als ein Vorhandensein eines Fluids oder als ein Vorhandensein eines Verbindungsmittels.

Erfüllt der Zustand die vorgegebene Bedingung nicht, bedeutet dies, dass der Leitungsabschnitt kein geschlossenes Fluidsystem ausbildet. Mit anderen Worten wird Fluid aus dem Leitungsabschnitt geführt. Das Fluid kann dabei entweder aus dem Leitungsabschnitt geführt werden, das heißt ein Abflussventil ist geöffnet, während gleichzeitig Fluid dem Leitungsabschnitt zugeführt werden kann, das heißt auch ein Einlassventil ist geöffnet. Alternativ kann das Fluid nur aus dem Leitungsabschnitt geführt werden, während kein Fluid dem Leitungsabschnitt zugeführt wird, mit anderen Worten wird der Leitungsabschnitt von dem Fluid geleert. Ein geschlossenes Fluidsystem wird nur ausgebildet, wenn der vom Bestimmungsmittel erfasste Zustand, das heißt das Vorliegen eines Fluids und/oder die Zusammensetzung eines Fluids und/oder die Position eines Verbindungsmittels eine vorgegebene Bedingung erfüllt.

Durch die anspruchsgemäße Blutbehandlungsvorrichtung wird die Unterbrechung der Behandlung möglichst kurzgehalten. Dies ist unter anderem dadurch erreicht, dass nur dann ein geschlossenes Fluidsystem ausgebildet wird, wenn der Fluidwert die vorgegebene Bedingung erfüllt. Damit wird verhindert, dass ein Druckhaltetest ausgeführt wird, obwohl die hierfür notwendigen Bedingungen nicht erfüllt sind. Somit kann die Durchführung eines Druckhaltetests zu einem frühen Zeitpunkt verhindert werden, wenn beispielsweise eine unphysiologische Flüssigkeit erfasst wird.

Im Ergebnis wird eine Durchführung des Druckhaltetests vermieden, wenn eine vorgegebene Bedingung nicht erfüllt ist, beispielsweise ein unphysiologisches Fluid vorliegt. Neben der Zeitersparnis wird hierdurch auch die Patientensicherheit gewährleistet. Unphysiologische Flüssigkeit wird nicht in der Blutbehandlungsvorrichtung gehalten. Die Wahrscheinlichkeit, dass unphysiologische Flüssigkeit den Patienten erreicht, wird weiter verringert.

Nach einer Weiterbildung der Blutbehandlungsvorrichtung kann die Blutbehandlungsvorrichtung weiter einen ersten Konzentratsensor zum Erfassen eines in der ersten Konzentratzufuhrleitung vorherrschenden ersten Konzentratwerts aufweisen, wobei das Bestimmungsmittel den ersten Konzentratsensor umfasst. Bei dieser Weiterbildung ist die vorgegebene erste Bedingung nur dann erfüllt, wenn der erste Konzentratwert eine erste Konzentratwertbedingung erfüllt. Zusätzlich oder alternativ kann die Blutbehandlungsvorrichtung einen Mischfluidsensor zum Erfassen eines in dem Fluidleitungssystem stromab der ersten Konzentratzufuhrleitung vorherrschenden Mischfluidwerts aufweisen, wobei das Bestimmungsmittel der Mischfluidsensor sein kann. Bei dieser zusätzlichen oder alternativen Weiterbildung ist die vorgegebene Bedingung nur dann erfüllt, wenn der Mischfluidwert eine Mischfluidwertbedingung erfüllt.

Mit anderen Worten kann das Bestimmungsmittel ein Konzentratsensor und/oder ein Mischfluidsensor sein. Das heißt, in einer ersten Alternative ist die vorgegebene Bedingung nur dann erfüllt, wenn sowohl der erste Konzentratwert als auch der Mischfluidwert jeweils eine vorgegebene Bedingung erfüllen. Die vorgegebene Bedingung für den Mischfluidwert und den Konzentratwert kann dabei jeweils eine andere Bedingung sein.

Das Bestimmungsmittel als Konzentratsensor und/oder Mischfluidsensor zum Erfassen eines Zustands des Fluidleitungssystems, hier einer Eigenschaft des Fluids, kann dabei ein Sensor sein, welcher eine oder mehrere chemische Zusammensetzungen des Fluids erfasst. Das Bestimmungsmittel kann dabei aber auch ein Indikator sein, welcher allein ein Vorhandensein des Fluids anzeigt ohne dabei eine Zusammensetzung des Fluids zu erfassen.

Erfasst das Bestimmungsmittel eine Eigenschaft des Fluids, und liegt diese erfasste Fluideigenschaft nicht in einem vorgegebenen Wertbereich, welcher in diesem Fall die vorgegebene Bedingung vorgibt, ist die vorgegebene Bedingung somit nicht erfüllt. Als Fluideigenschaft im Sinne dieser Erfindung ist die Zusammensetzung des Fluids zu verstehen. Dabei kann die Zusammensetzung einen Bereich umfassen, welcher bei null beginnt, wobei null bedeutet, dass kein Fluid vorliegt.

In einer zweiten Alternative erfüllt der Zustand die vorgegebene Bedingung nur, wenn sowohl der Konzentratwert als auch der Mischfluidwert eine jeweils vorgegebene Bedingung erfüllen. Mit anderen Worten kann die Situation auftreten, dass der Zustand in der zweiten Alternative die vorgegebene Bedingung nicht erfüllen, obwohl der Konzentratwert oder der Mischfluidwert die jeweils vorgegebene Bedingung erfüllt. Erfüllt der Konzentratwert und/oder der Mischfluidwert eine jeweils vorgegebene Bedingung nicht, bedeutet dies im Sinne der Erfindung, dass der Zustand des Fluidleitungssystems, erfasst durch den Konzentratsensor oder Mischfluidsensor, die vorgegebene Bedingung nicht erfüllt.

Der erste Konzentratwert im Sinne dieser Erfindung meint eine Eigenschaft der Zusammensetzung einer Konzentratlösung, welche in der ersten Konzentratzufuhrleitung vorliegt. Die Konzentratlösung ist dabei ein Fluid, welches mit einem weiteren, der Blutbehandlungsvorrichtung zugeführten Fluid zusammengeführt wird, und damit ein Mischfluid ausbildet. Der Mischfluidwert im Sinne dieser Erfindung meint damit eine Eigenschaft der Zusammensetzung des Mischfluids. Das Mischfluid wird erfindungsgemäß erst stromab der Konzentratzufuhrleitung gebildet.

Durch das separate Erfassen von Eigenschaften des Fluids an verschiedenen Punkten in dem Fluidleitungssystem wird die Wahrscheinlichkeit erhöht, dass eine unphysiologische Flüssigkeit schnell erfasst wird. Zeitgleich wird durch das Erfassen der Fluideigenschaft an verschiedenen Punkten die Wahrscheinlichkeit erhöht, dass eine unphysiologische Flüssigkeit erkannt wird.

Nach einer Weiterbildung kann die Blutbehandlungsvorrichtung weiter ein Verbindungsmittel zum Überführen der ersten Konzentratlösung in die erste Konzentratzufuhrleitung sowie eine Kammer aufweisen, in welche das Verbindungsmittel einbringbar ist. Weiter kann die Blutbehandlungsvorrichtung einen Verbindungsmittelsensor zum Erfassen einer Position des Verbindungsmittels aufweisen. Dabei umfasst bei dieser Weiterbildung das Bestimmungsmittel den Verbindungsmittelsensor und die vorgegebene Bedingung ist nur dann erfüllt, wenn die Position des Verbindungsmittels eine Positionsbedingung erfüllt.

Ein Verbindungsmittel im Sinne der Erfindung ist ein Mittel, durch welches ein Überführen eines Fluids ermöglicht wird. Dabei ist das Verbindungsmittel ein rohrförmiges, sowie relativ zu der Kammer bewegliches Element. Mit anderen Worten kann das Verbindungsmittel eine flexible Verbindung, etwa eine Schlauchanordnung, mittels welcher ein Fluid förderbar ist, oder ein starres Verbindungselement, etwa ein Ansaugstab, sein. Das Fluid kann dabei sowohl in die Konzentratzufuhrleitung durch Aufbringen von Überdruck gedrückt, durch eine Pumpe gefördert oder durch Erzeugen von Unterdruck gesogen werden.

Ein erfindungsgemäßer Verbindungsmittelsensor kann die Position des Verbindungsmittels erfassen. Dabei kann der Verbindungsmittelsensor die Position des Verbindungsmittels positiv als ein Vorhandensein des Verbindungsmittels erfassen. Ein Erfassen der Position im Sinne dieser Erfindung kann aber auch ein negatives Erfassen bedeuten, so dass der Verbindungsmittelsensor erfasst, dass das Verbindungsmittel nicht vorhanden ist. Der Verbindungsmittelsensor kann sowohl ein Kontaktsensor, Lichtsensor oder Magnetsensor sein.

Eine Kammer im Sinn der Erfindung ist ein Hohlraum, welcher in der Blutbehandlungsvorrichtung ausgebildet ist, und welcher eine Öffnung zur Umgebung aufweist. Über diese Öffnung kann das Verbindungsmittel in die Kammer eingebracht werden. Die Kammer ist in ihren Abmessungen damit an das Verbindungsmittel derart angepasst, dass ein Herausfallen des Verbindungsmittels verhindert wird. Insbesondere kann etwa ein Rastmechanismus an der Kammer ausgebildet sein, welcher mit dem Verbindungsmittel wechselwirkt.

Wird mit Hilfe des Verbindungsmittelsensors erfasst, dass das Verbindungsmittel nicht an einer Position ist, an welcher physiologische Flüssigkeit gefördert werden kann, kann somit noch vor einem Erfassen der unphysiologischen Flüssigkeit mittels eines Fluidsensors, dem Mischfluidsensor oder Konzentratsensor, der Zustand erkannt werden, in welchem eine unphysiologische Flüssigkeit in der Blutbehandlungsvorrichtung vorliegt.

Durch diese Weiterbildung wird die Wahrscheinlichkeit weiter erhöht, dass eine unphysiologische Flüssigkeit früh erkannt wird. Die Durchführung eines Druckhaltetest bei unphysiologischen Bedingungen, beispielsweise dem Vorliegen einer unphysiologische Flüssigkeit, kann verhindert werden. Damit ergibt sich insbesondere der Vorteil der Zeitersparnis, da der Druckhaltetest möglichst früh abgebrochen wird, oder bei entsprechender Steuerung ein zeitintensiver Spülvorgang verhindert werden kann.

Nach einer Weiterbildung kann die Blutbehandlungsvorrichtung weiter ein Mittel zum Erfassen eines Konzentratversorgungsmodus aufweisen. Bei dieser Weiterbildung ist die vorgegebene Bedingung nur dann erfüllt, wenn die Position des Verbindungsmittels die Positionsbedingung erfüllt und wenn der erfasste Konzentratversorgungsmodus ein Konzentratversorgungsmodus ist, in dem über das Verbindungsmittel in der erfassten Position die Konzentratzufuhr erfolgt.

Ein Konzentratversorgungsmodus im Sinne der Erfindung gibt den Modus der Konzentratversorgung der Blutbehandlungsvorrichtung an. Eine Konzentratversorgung kann dabei über eine in flüssiger Form bereitgestellte Konzentratlösung oder über ein Trockenkonzentrat erfolgen. Bei dem ersten Konzentratversorgungsmodus wird die für eine Behandlung notwendige Menge an Konzentrat in flüssiger Form in einem Konzentratbehälter der Blutbehandlungsvorrichtung zugestellt. Das Konzentrat ist damit in einer Lösung bereits vorbereitet.

Mit anderen Worten kann der erste Konzentratversorgungsmodus ein Modus sein, bei welchem die Zufuhr der ersten Konzentratlösung in die erste Konzentratzufuhrleitung über das Verbindungsmittel erfolgt, wobei das Verbindungsmittel in dem ersten Konzentratversorgungsmodus zur Zufuhr der ersten Konzentratlösung nicht in der Kammer eingebracht ist.

Bei einem anderen Konzentratversorgungsmodus wird das in flüssiger Form bereitgestellte Konzentrat über eine Zentralversorgung zugeführt. Hierfür ist ein Zentralversorgungsanschluss an der Blutbehandlungsvorrichtung vorgesehen. Über den Zentralversorgungsanschluss wird eine Zentralversorgungsleitung angeschlossen. Der Konzentratspeicher ist bei diesem Konzentratversorgungsmodus entfernt von der Blutbehandlungsvorrichtung, etwa in einem anderen Raum, vorgesehen.

Bei einer weiteren Alternative der Konzentratversorgung kann das Konzentrat als Trockenkonzentrat in einem Konzentratbeutel der Blutbehandlungsvorrichtung zugeordnet werden. Beispielsweise kann das Trockenkonzentrat in einem Konzentratbeutel an der Blutbehandlungsvorrichtung angehängt werden.

Wird bei der Blutbehandlungsvorrichtung neben der Position des Verbindungsmittels zudem der Konzentratversorgungsmodus erfasst, wird damit die Wahrscheinlichkeit erhöht, dass tatsächlich eine unphysiologische Flüssigkeit vorliegt. Verringert wird damit die Wahrscheinlichkeit für das Eintreten des Falls, dass das Bestimmungsmittel erfasst, dass das Fluidleitungssystem die vorgegebene Bedingung nicht erfüllt, obwohl keine unphysiologische Flüssigkeit vorliegt. Mit anderen Worten wird ein unnötiges Verhindern des Druckhaltetest vermieden.

Nach einer Weiterbildung kann die Blutbehandlungsvorrichtung weiter eine zweite Konzentratzufuhrleitung zum Zuführen einer zweiten Konzentratlösung sowie einen zweiten Konzentratsensor zum Erfassen eines zweiten Konzentratwerts in der zweiten Konzentratzufuhrleitung aufweisen. Bei dieser Weiterbildung umfasst das Bestimmungsmittel den zweite Konzentratsensor, und die vorgegebene Bedingung ist nur dann erfüllt, wenn der zweite Konzentratwert eine zweite Konzentratwertbedingung erfüllt.

Die zweite Konzentratzufuhrleitung im Sinne der Erfindung ist eine weitere Zufuhrleitung mittels welcher ein Fluid, genauer eine Konzentratlösung, zugeführt wird. Dabei kann die zweite Konzentratzufuhrleitung zuerst mit der ersten Konzentratzufuhrleitung zusammenführen, um anschließend einem weiteren Fluid, etwa einem Permeat zugeführt zu werden, und das Mischfluid auszubilden. Alternativ kann die erste oder die zweite Konzentratzufuhrleitung zuerst mit dem weiteren Fluid zusammengeführt werden, um anschließend mit der ersten oder zweiten Konzentratlösung zusammengeführt zu werden. Alternativ können die erste und zweite Konzentratlösung gleichzeitig, das heißt über einen Mischpunkt, zugeführt werden und so das Mischfluid bilden.

Die vorgegebene Bedingung ist bei dieser Weiterbildung erfüllt, wenn der zweite Konzentratwert eine zweite Konzentratwertbedingung erfüllt. Die zweite Konzentratwertbedingung ist eine Bedingung für eine Zusammensetzung des zweiten Konzentrats. Die zweite Konzentratwertbedingung kann dabei von der ersten Konzentratwertbedingung verschieden sein.

Nach einer Weiterbildung kann der Verbindungsmittelsensor einen Magnetsensor oder einen Kontaktsensor, insbesondere einen Hall-Sensor, oder einen mechanischen Schalter aufweisen.

Nach einer Weiterbildung kann an der Blutbehandlungsvorrichtung ein Verbindungsmittelsensor an der Kammer der Blutbehandlungsvorrichtung angeordnet sein. Zusätzlich oder alternativ kann der Verbindungsmittelsensor derart ausgebildet sein, dass er an einem der Blutbehandlungsvorrichtung zustellbarem Konzentratbehälter anbringbar ist.

Der Verbindungsmittelsensor kann im Sinne der Erfindung an der Kammer der Blutbehandlungsvorrichtung angeordnet sein. Mit anderen Worten erfasst der an der Kammer der Blutbehandlungsvorrichtung angeordnete Verbindungsmittelsensor, ob das Verbindungsmittel in der Kammer eingebracht ist, beziehungsweise, ob sich das Verbindungsmittel zumindest teilweise in der Kammer der Blutbehandlungsvorrichtung befindet. Dabei kann der Verbindungsmittelsensor ein Kontaktsensor, etwa ein Hall-Sensor, sein.

Alternativ oder zusätzlich kann sich der Verbindungsmittelsensor an einem Ort entfernt von der Kammer befinden. Damit kann der Verbindungsmittelsensor erfassen, dass sich das Verbindungsmittel entfernt von der Kammer, und damit nicht in der Kammer befindet.

Von der erfindungsgemäßen Lösung mit umfasst sind damit Sensoranordnungen, welche sowohl an dem Verbindungsmittel als auch an der Kammer angeordnet sind. Damit kann ein Magnet an dem Verbindungsmittel oder an einem Ort entfernt von der Blutbehandlungsvorrichtung, etwa einem Konzentratbehälter, welcher der Blutbehandlungsvorrichtung zugestellt wird, angebracht sein. Der entsprechende Magnetsensor kann als Gegenstück entsprechend an dem Konzentratbehälter oder dem Verbindungsmittel angeordnet sein. Umfasst als Verbindungsmittelsensor ist ebenso ein Hall-Sensor, welcher an der Kammer der Blutbehandlungsvorrichtung oder an einem Ort entfernt von der Blutbehandlungsvorrichtung, etwa einem Konzentratbehälter, angeordnet ist.

Nach einer Weiterbildung kann der erste und/oder der zweite Konzentratsensor ein Leitwertsensor oder ein Leitfähigkeitssensor oder ein Ultraschallsensor sein. Zusätzlich oder alternativ kann der Mischfluidsensor ein Leitwertsensor oder ein Leitfähigkeitssensor oder ein Ultraschallsensor sein. Für die Konzentratsensoren können andere Arten von Konzentratsensoren verwendet werden als für den Mischfluidsensor. Damit ist es möglich, die Systemsicherheit auf Grund von verschiedenen Sensorarten und damit Kontroll- beziehungsweise Steuermechanismen zu erhöhen.

Nach einer Weiterbildung kann die Blutbehandlungsvorrichtung weiter einen Leitungszweig aufweisen. Der Leitungszweig ist dabei parallel zu zumindest einem Teilabschnitt des Leitungsabschnitts ausgebildet, so dass bei einem Fluidfluss über den Leitungsabschnitt ein Fluidfluss über den Leitungszweig verhinderbar ist. Zudem ist in dem Leitungszweig ein Dialysator angeordnet.

Der Leitungszweig im Sinne der Erfindung ist ein Fluidleitungsabschnitt, welcher parallel zu einem Teilabschnitt des Fluidleitungssystems verläuft. Dabei ist der Leitungszweig parallel zu einem Teilabschnitt des Leitungsabschnitts ausgebildet, so dass bei einem Fluidfluss über den Leitungsabschnitt kein Fluidfluss über den Leitungsabschnitt stattfindet.

Nach dieser Weiterbildung der Blutbehandlungsvorrichtung weist die Blutbehandlungsvorrichtung einen Dialysator in dem Leitungszweig auf. In diesem Fall ist die Blutbehandlungsvorrichtung als Dialysemaschine ausgebildet.

Durch das Ausbilden des Dialysators in dem Leitungszweig kann die Sicherheit erhöht werden. Insbesondere kann der Dialysator von dem Fluidfluss entkoppelt werden, indem das Fluid über den zu dem Leitungszweig parallel verlaufenden Teil des Leitungsabschnitts geführt wird. Zudem kann der Leitungsabschnitt als ein geschlossenes System ausgebildet werden. In einem geschlossenen System, in welchem kein Zu- und Abfluss von Fluid stattfindet, kann durch Überwachen des Druckverlaufs in diesem System die Funktionsweise der Systemkomponenten, beispielsweise von Ventilen, überprüft werden.

Nach einer Weiterbildung weist die Blutbehandlungsvorrichtung weiter Absperrelemente in dem Fluidleitungssystem auf. Dabei kann die Steuereinheit derart konfiguriert sein, dass durch Ansteuern zumindest der Absperrelemente der Leitungsabschnitt als ein geschlossenes Fluidsystem ausgebildet wird.

Nach einer Weiterbildung der Erfindung weist die Blutbehandlungsvorrichtung weiter einen Drucksensor zum Erfassen eines Druckwerts in dem Leitungsabschnitt auf. Dabei ist die Steuereinheit bei dieser Weiterbildung derart konfiguriert, nur dann, wenn der von dem Bestimmungsmittel erfasste Zustand die vorgegebene Bedingung erfüllt, in dem Leitungsabschnitt einen Druckhaltetest in dem Leitungsabschnitt durchzuführen. Bei diesem Druckhaltetest wird Fluid in dem Leitungsabschnitt mit Druck, bevorzugt von 600 bis 800 mmHg, noch bevorzugter von 700 bis 750 mmHg beaufschlagt, und während eines vorgegebenen Zeitraums werden die Druckwerte in dem Leitungsabschnitt erfasst und aus einer Änderung der Druckwerte kann auf eine Leckage in dem Leitungsabschnitt geschlossen werden.

Der Leitungsabschnitt kann als ein geschlossenes System ausgebildet werden. In dem geschlossenen System kann ein Druckhaltetest durchgeführt werden. Ein Druckhaltetest im Sinne der Erfindung ist ein Verfahren, bei welchem während einer Behandlung in periodischen Zeitabständen der Dialysator für jeweils ein kurzes Zeitintervall, beispielsweise 8 Sekunden, vom Dialysierflüssigkeitskreislauf abgetrennt wird. Der Druckverlauf in diesem kurzen Zeitintervall wird durch Detektion von Signalen mittels zumindest eines Druckaufnehmers erfasst. Aus den erfassten Druckwerten, insbesondere aus dem Verlauf der Druckwerte, kann anschließend auf den Zustand des Systems und Systemkomponenten geschlossen werden und eine mögliche Leckage identifiziert werden. Die während des Druckhaltetests in dem Dialysierflüssigkeitskreislauf vorliegenden Drücke, besonders bevorzugt + 725mmHg, liegen dabei über den während der Behandlung vorliegenden Drücken.

Nach einer Weiterbildung kann die Blutbehandlungsvorrichtung zumindest eine erste Konzentratpumpe zum Fördern der ersten Konzentratlösung aufweisen.

Die Konzentratpumpe zum Fördern der ersten Konzentratlösung kann in der ersten Konzentratzufuhrleitung angeordnet sein. Alternativ kann die Konzentratpumpe auch entfernt von der ersten Konzentratzufuhrleitung angeordnet sein, und beispielsweise durch Erzeugen von Unterdruck in der ersten Konzentratzufuhrleitung die erste Konzentratlösung fördern.

Es wird weiter ein Verfahren zum Steuern eines Fluidflusses in einer Blutbehandlungsvorrichtung mit den zuvor genannten Vorteilen vorgeschlagen.

Das Verfahren zum Steuern eines Fluidflusses in einer Blutbehandlungsvorrichtung, wobei die Blutbehandlungsvorrichtung ein Fluidleitungssystem zum Leiten eines Fluidflusses mit einem Leitungsabschnitt, der als ein geschlossenes Fluidsystem ausbildbar ist, und zumindest einer ersten Konzentratzufuhrleitung zum Zuführen einer ersten Konzentratlösung, aufweist, weist die folgenden Schritte auf: Detektieren eines Zustands des Fluidleitungssystems mit einem Bestimmungsmittel; und Abgleichen des Zustands mit einer vorgegebenen Bedingung.

Dabei ist das Verfahren gekennzeichnet durch ein Zulassen, dass der Leitungsabschnitt ein geschlossenes Fluidsystem nur dann ausbildet, wenn der Zustand des Fluidleitungssystems die vorgegebene Bedingung erfüllt.

Dabei kann eine Weiterbildung des Verfahrens vorgesehen sein, wobei die Blutbehandlungsvorrichtung einen ersten Konzentratsensor als Bestimmungsmittel zum Erfassen eines in der ersten Konzentratzufuhrleitung vorherrschenden ersten Konzentratwerts aufweist, und wobei die vorgegebene Bedingung nur dann erfüllt ist, wenn der Konzentratwert eine Konzentratwertbedingung erfüllt und/oder die Blutbehandlungsvorrichtung einen Mischfluidsensor als Bestimmungsmittel zum Erfassen eines Mischfluidwerts in dem Fluidleitungssystem stromabwärts der Konzentratzufuhrleitung aufweist, und wobei die vorgegebene Bedingung nur dann erfüllt ist, wenn der Mischfluidwert eine Mischfluidwertbedingung erfüllt.

Dabei kann eine Weiterbildung zusätzlich oder alternativ des Verfahrens vorgesehen sein, wobei die Blutbehandlungsvorrichtung eine Kammer und ein in die Kammer einbringbares Verbindungsmittel sowie einen Verbindungsmittelsensor als Bestimmungsmittel zum Erfassen einer Position des Verbindungsmittels aufweist, weiter aufweisend Detektieren einer Position des Verbindungsmittels durch den Verbindungsmittelsensor, wobei die vorgegebene Bedingung nur dann erfüllt ist, wenn die Position des Verbindungsmittels eine Positionsbedingung erfüllt.

Insbesondere wird durch diese anspruchsgemäße Lösung eine unphysiologische Flüssigkeit möglichst früh erkannt, etwa bevor diese den Dialysierflüssigkeitskreislauf erreicht hat. Die Unterbrechung der Behandlung kann damit möglichst geringgehalten werden, indem der Druckhaltetest unmittelbar beendet wird, beziehungsweise der Beginn eines Druckhaltetests vermieden wird, sobald eine unphysiologische Flüssigkeit erkannt wird. Im Ergebnis kann ein Durchführen eines Druckhaltetest vermieden werden, wenn unphysiologische Bedingungen vorliegen, das heißt die Bedingungen für den Fluidwert nicht erfüllt sind. Neben dem zeitlichen Vorteil wird zudem die Patientensicherheit gewährleistet, da kein geschlossenes System ausgebildet wird, in welchem unphysiologische Flüssigkeit mit Druck beaufschlagt wird.

Zudem kann die Steuerung derart gestaltet werden, dass ein zeitaufwendiger Spülvorgang des Dialysierflüssigkeitskreislaufs verhindert wird, da die unphysiologische Flüssigkeit zu einem möglichst frühen Zeitpunkt, beispielsweise vor Erreichen des Dialysierflüssigkeitskreislauf, erkannt wird.

Die vorstehend beschriebenen Merkmale und Funktionen der vorliegenden Erfindung sowie weitere Aspekte und Merkmale werden nachfolgend anhand einer detaillierten Beschreibung von bevorzugten Ausführungsformen unter Bezugnahme auf die beigefügten Figuren weiter beschrieben. In den Figuren sind gleiche Merkmale/Elemente und Merkmale/Elemente mit der gleichen Funktion mit den gleichen Bezugszeichen gekennzeichnet.

### Kurzbeschreibung der Zeichnungen

Hierbei zeigt:
- Fig. 1: ein Schaubild einer Blutbehandlungsvorrichtung;
- Fig. 2: einen Ausschnitt einer Konzentratversorgungsanordnung der Blutbehandlungsvorrichtung;
- Fig. 3: ein Ablaufdiagramm eines Verfahrens zum Steuern eines Fluidflusses in einer Blutbehandlungsvorrichtung.

### Detaillierte Beschreibung eines Ausführungsbeispiels

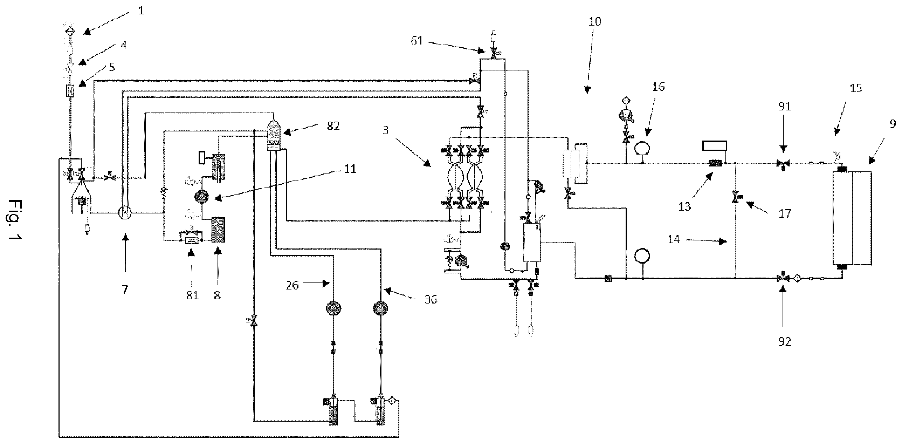

Bezugnehmend auf Fig. 1 wird nachstehend eine erste Ausführungsform erläutert werden. Dabei zeigt Fig. 1 ein vereinfachtes Schaubild einer Blutbehandlungsvorrichtung. In dem in Fig. 1 gezeigten Ausführungsbeispiel ist die Blutbehandlungsvorrichtung eine Dialysemaschine.

Bei der in Fig. 1 gezeigten Blutbehandlungsvorrichtung durchströmt das zu behandelnde Blut in einem extrakorporalen Blutkreislauf eine Blutkammer eines Dialysators 9. Eine Dialysierflüssigkeit durchströmt einen Dialysierflüssigkeitskreislauf und im Gegenstromprinzip eine Dialysierflüssigkeitskammer des Dialysators 9. Dabei sind die Blutkammer sowie die Dialysierflüssigkeitskammer durch eine semipermeable Membran getrennt.

Durch die semipermeable Membran treten aus dem Blut zu entfernende Stoffe in die Dialysierflüssigkeit über und werden so durch die Dialysierflüssigkeit, nun Dialysat genannt, abgeführt. Gleichzeitig kann über einen Druckgradienten eine überschüssige Flüssigkeitsmenge aus dem Blut ultrafiltriert werden. Die zu entfernende Flüssigkeitsmenge wird mit Hilfe einer Ultrafiltrationspumpe gefördert. Zur Blutreinigung eines Patienten wird diesem über eine arteriovenöse Fistel mittels eines Shunts Blut entnommen und in den extrakorporalen Blutkreislauf geleitet. Die Förderung des Blutes erfolgt dabei mit Hilfe einer hier nicht dargestellten Blutpumpe. Das gereinigte Blut verlässt den Dialysator 9 und wird dem Patienten anschließend wieder zugeführt.

Die Flüssigkeitsversorgung der Blutbehandlungsvorrichtung erfolgt über einen Dialysewasseranschluss 1, ein nachgeschaltetes Druckminderventil 4, welches den Druck beispielsweise auf etwa 0,5 bar reduziert, sowie eine Zulaufdrossel 5. Über den Dialysewasseranschluss 1 wird Permeat, das heißt enthärtetes und filtriertes Wasser, zugeführt. In dem hier beschriebenen Ausführungsbeispiel der Blutbehandlungsvorrichtung als Dialysemaschine ist Permeat die Basisflüssigkeit.

Nach dem Passieren des Dialysators 9 wird das Dialysat über eine Dialysierflüssigkeitsabführleitung und ein Abflussventil 61 einem Abfluss zugeführt. Dabei kann die Erwärmung der frischen Dialysierflüssigkeit über einen Wärmetauscher 7 durch das Dialysat stattfinden, um anschließend etwa mittels einer Heizspirale oder eines Heizstabs weiter erwärmt zu werden. Zudem wird das Permeat einer Entgasung in einer Entgasungskammer 8 unterzogen. Um die Lösung von Luft zu fördern, wird das Permeat hierfür mittels einer Entgasungsdrossel 81 einem Unterdruck ausgesetzt. Durch die Temperaturerhöhung und Druckverringerung kann damit Luft in Blasenform über einen nachgeschalteten Luftabscheider 82 entweichen.

Das Permeat wird mit Hilfe einer Fluidpumpe 11 gefördert. Die Fluidpumpe 11 kann dabei beispielsweise als Zahnradpumpe, Membranpumpe oder ähnlichem ausgebildet sein. Ist die Fluidpumpe 11 als Zahnradpumpe ausgebildet, ist ein Bypass um die Zahnradpumpe ausgebildet, so dass die Zahnradpumpe bei einem Verhindern des Fluidflusses nicht angehalten werden muss. Das Dialysat wird, wie zuvor beschrieben, über eine Flusspumpe und einer sich in Strömungsrichtung anschließender Bilanzkammer 3 einem Abfluss zugeführt.

Nach der Entgasung der Basisflüssigkeit, hier des Permeats, wird durch die Zumischung mindestens einer Konzentratlösung das Mischfluid, hier die Dialysierflüssigkeit erzeugt. Zur Bereitstellung der frischen Dialysierflüssigkeit wird somit Permeat, zugeführt über den Dialysewasseranschluss 1, sowie beispielsweise zwei Konzentratlösungen, etwa einer Bicarbonatkonzentratlösung und einer Säurekonzentratlösung, zugeführt beispielsweise aus hier nicht dargestellten Konzentratbehältern vermischt.

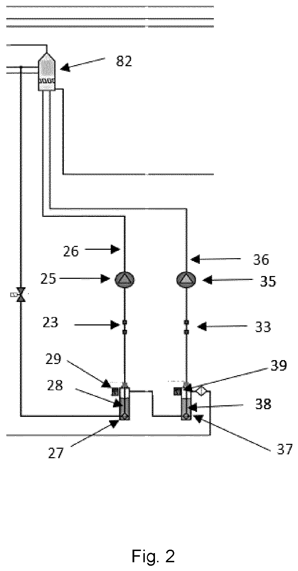

Wie in Fig. 2 dargestellt ist, können die Konzentratlösungen über Konzentratpumpen 25, 35 gefördert werden. Die Konzentratpumpen 25, 35 können beispielsweise als Hubkolbenpumpen, Membranpumpen oder Zahnradpumpen ausgebildet sein. Die Proportionierung, das heißt die Mischung von Säurekonzentrat und Bicarbonat mit Permeat in einem vorbestimmten Verhältnis, kann volumetrisch oder leitfähigkeitsgesteuert erfolgen. Bei der in diesem Ausführungsbeispiel gezeigten volumetrischen Proportionierung wird das zugeführte Volumen über eine getaktete Zuführung mittels der Konzentratpumpen 25, 35, beispielsweise Hubkolbenpumpen erreicht.

Alternativ kann die Proportionierung aber auch leitfähigkeitsgesteuert erfolgen, wobei hier die Proportionierung mittels Leitfähigkeitssensoren gesteuert wird. Dabei wird die Zuführung von Konzentrat erhöht, bis die gewünschte Leitfähigkeit erreicht ist. Die durch die Vermischung entstandene Dialysierflüssigkeit durchströmt anschließend einen Teil der Bilanzkammer 3 und gelangt damit in den Dialysierflüssigkeitskreislauf. Die Bilanzkammer 3 bilanziert dabei zwischen der frischen Dialysierflüssigkeit und der verbrauchten Dialysierflüssigkeit, dem Dialysat. Dem Dialysator 9 vorgeschaltet ist ein Mischfluidsensor 13, um die richtige Zusammensetzung der Dialysierflüssigkeit zu überprüfen. Dem Mischfluidsensor 13, beispielsweise als Leitfähigkeitssensor ausgebildet, ist ein Bypass-Ventil 17 nachgeschaltet.

Detektiert der Mischfluidsensor 13 während der Dialysebehandlung eine unphysiologische Flüssigkeit, das heißt eine Flüssigkeit, welche einer vorgegebenen Bedingung, beispielsweise einer vorgegebenen Leitfähigkeit nicht erfüllt, wird das Bypass-Ventil 17 geöffnet.

Zur Ausbildung des Bypasses, das heißt der Verhinderung eines Fluidflusses über einen Leitungszweig 15 durch den Dialysator 9, werden Absperrelemente 91, 92 betätigt. Dabei werden insbesondere ein Dialysatorzulaufventil 91 sowie ein Dialysatorablaufventil 92, welche den Zu- und Ablauf der Dialysierflüssigkeit zum Dialysator 9 kontrollieren, geschlossen. Die Dialysierflüssigkeit strömt somit über einen Leitungsabschnitt 14. Die Ventile können dabei als Magnetventile ausgebildet sein. Im Ergebnis wird verhindert, dass unphysiologische Flüssigkeit zu dem Dialysator 9 gelangt. Die Sicherheit des Patienten wird gewährleistet.

Neben der Überwachung der richtigen Zusammensetzung der Dialysierflüssigkeit kann zur Gewährleistung der Sicherheit des Patienten auch die Funktionsfähigkeit der Blutbehandlungsvorrichtung, beispielsweise auf das Vorliegen einer Leckage, überprüft werden. Um mögliche Leckagen in dem System zu identifizieren wird ein Druckhaltetest durchgeführt. Im Rahmen dieses Druckhaltetests können mittels Signalüberwachung von zumindest einem Drucksensor 16 Abweichungen von einem stabilen Zustand erfasst werden.

Der Druckhaltetest wird während der Dialyse in periodischen Zeitabständen, beispielsweise alle 12,5 Minuten, durchgeführt. Hierfür wird der Dialysator 9 für ein bestimmtes Zeitintervall, beispielsweise 8 Sekunden, von dem Dialysierflüssigkeitskreislauf abgetrennt. Damit wird der Leitungsabschnitt 14 während der Durchführung des Druckhaltetests als ein geschlossenes Fluidsystem ausgebildet. Zur Ausbildung dieses geschlossenen Fluidsystems steuert die Steuereinheit die Absperrelemente 91, 92 derart an, dass diese den Fluidfluss sperren. Zur Ausbildung des geschlossenen Fluidsystems wird zusätzlich die Bilanzkammer 3 in einem Zustand gehalten, das heißt es findet kein Fluidfluss über die Bilanzkammer, das heißt kein Umschalten der Bilanzkammer 3 statt.

Wenn der Dialysator 9 vom Dialysierflüssigkeitskreislauf abgetrennt wird, befindet sich die Blutbehandlungsvorrichtung somit im Bypass-Betrieb. Wie zuvor beschrieben sind das Dialysatorzulaufventil 91 sowie Dialysatorablaufventil 92 geschlossen, während das Bypass-Ventil 17 geöffnet ist. Im Ergebnis wird der Leitungszweig 15 von dem restlichen Fluidleitungssystem 10 durch Schließen der entsprechenden Ventile abgetrennt. Mit anderen Worten findet nach dem Schließen der Ventile kein Fluidfluss zwischen dem Leitungszweig 15 und dem restlichen Fluidleitungssystem 10 statt. Um eine möglichst vollständige Prüfung zu erreichen, erfolgt zwischen zwei aufeinanderfolgenden Druckhaltetests ein Wechsel der zu dem Leitungsabschnitt 14 gehörenden Bilanzkammerhälfte.

Dieser Leitungsabschnitt 14 bildet in diesem Fall ein in sich geschlossenes System, bei welchem ein stabiler Druck zu erwarten ist. Der während des Druckhaltetests in dem Leitungsabschnitt 14 vorherrschende Druck ist dabei im Vergleich zu dem Behandlungsdruck erhöht. Um mögliche Leckagen zu erkennen, wird der Druckverlauf während des Druckhaltetests erfasst. Wird ein Druckabfall erkannt, kann auf das Vorhandensein einer Leckage geschlossen werden.

Vor Beginn des Druckhaltetests wird aus dem Zufuhrsystem, und damit auch über die Konzentrationszufuhrleitung 26, 36 Dialysierflüssigkeit zugeführt, bis der gewünschte Druck erreicht ist. Während der Zufuhr der Dialysierflüssigkeit in den Leitungsabschnitt 14 findet kein Fluidabfluss statt, so dass Druck aufgebaut wird. Wird nun mittels des Mischfluidsensors 13, das Vorliegen einer Bedingung, beispielsweise eine Leitfähigkeit gemessen, welche nicht dem erwarteten Wert entspricht, kann auf das Vorliegen einer unphysiologischen Flüssigkeit im Dialysierflüssigkeitskreislauf geschlossen werden.

Wird eine unphysiologische Flüssigkeit erkannt, schaltet die Blutbehandlungsvorrichtung, wie oben beschrieben, in den Bypass-Betrieb um zu verhindern, dass unphysiologische Flüssigkeit den Dialysator 9 erreicht. Nach der Detektion einer unphysiologischen Flüssigkeit kann der Dialysierflüssigkeitskreislauf in einem Reinigungsprogramm gespült werden. Damit kann verhindert werden, dass unphysiologische Flüssigkeit, welche im Dialysierflüssigkeitskreislauf vorhanden ist, in den Dialysator 9 gelangt.

Durch die erfindungsgemäße Blutbehandlungsvorrichtung wird das Ausbilden eines geschlossenen Systems, beispielsweise zur Durchführung des Druckhaltetest verhindert, sobald eine unphysiologische Flüssigkeit erkannt wird. Damit wird der Druckhaltetest zu einem möglichst frühen Zeitpunkt verhindert, beziehungsweise ein bereits begonnener Druckhaltetest abgebrochen. Im Ergebnis kann die Unterbrechung der Behandlung insgesamt möglichst geringgehalten werden.

Wie in Fig. 2 dargestellt ist, weist die Blutbehandlungsvorrichtung für die Zufuhr von Konzentrat Konzentratzufuhrleitungen 26, 36 auf. Über diese Konzentratzufuhrleitungen 26, 36 wird die entsprechende Konzentratmenge dem Permeat über die jeweilige Konzentratpumpe 25, bzw. 35 zugeführt und so die gewünschte Zusammensetzung der Dialysierflüssigkeit erreicht.

Das jeweilige Konzentrat wird als Konzentratlösung, das heißt in flüssiger Form, dem Permeat zugeführt. In den jeweiligen Konzentratzufuhrleitungen 26, 36 sind jeweils Konzentratsensoren 23, 33 angeordnet. Mittels dieser Konzentratsensoren 23, 33 kann die Zuführung des Konzentrats, bzw. die Zusammensetzung der Konzentratlösung überwacht werden. Die Überwachung der Konzentratzufuhr über Konzentratzufuhrleitungen 26, 36 kann beispielsweise mittels Leitwertsensoren oder Leitfähigkeitssensoren erfolgen.

Die Konzentratversorgung der Blutbehandlungsvorrichtung kann aus der Blutbehandlungsvorrichtung zugestellten Konzentratbehältern mittels in diese eingesteckte Verbindungsmittel 28, 38 erfolgen. In der in Fig. 2 dargestellten Anordnung sind die Verbindungsmittel 28, 38 in Kammern 27, 37 der Blutbehandlungsvorrichtung eingesteckt. Diese Konfiguration, in welcher sich die Verbindungsmittel 28, 38 in der Kammer 27, 37 befinden, wird für einen Spülvorgang der Blutbehandlungsvorrichtung angenommen. Zudem unterscheidet sich, wie nachfolgend beschrieben wird, die Position der Verbindungsmittel 28, 38 je nach Konzentratversorgungsmodus. Zur Positionserfassung sind Verbindungsmittelsensoren 29, 39 in der in Fig. 2 gezeigten Ausführungsform an den Kammern 27, 37 der Blutbehandlungsvorrichtung angebracht.

In den in Fig. 2 nicht dargestellten Konzentratbehältern ist eine entsprechende Konzentratlösung in flüssiger Form für die Dialysebehandlung vorbereitet. Diese Art der Konzentratversorgung wird hier als erster Konzentratversorgungsmodus (KVM) verstanden. Alternativ kann die Konzentratversorgung aus an der Blutbehandlungsvorrichtung befestigten Beuteln mit Trockenkonzentrat oder als zentrale Konzentratversorgung ausgestaltet sein.

Auch können Varianten vorgesehen sein, bei welchem die Konzentratversorgung für die jeweiligen Konzentrate auf unterschiedliche Weise geschieht. Beispielsweise kann die Konzentratversorgung von Bicarbonat als durch in Beutel gefülltes Trockenkonzentrat ausgestaltet sein, während das Säurekonzentrat in flüssiger Form in einem Konzentratbehälter der Blutbehandlungsvorrichtung zugestellt wird.

Im ersten Fall, in welchem die Konzentratversorgung aus in flüssiger Form bereitgestellte Konzentratlösung über Konzentratbehälter erfolgt, werden Verbindungsmittel 28, 38, beispielsweise Ansaugstäbe, in die jeweiligen Konzentratbehälter eingesteckt. Diese Verbindungsmittel 28, 38 führen die Konzentratlösung über die Konzentratzufuhrleitung 26, 36 dem Permeat zu.

Für die zweite Alternative der Konzentratversorgung mittels Trockenkonzentrat wird das jeweilige Verbindungsmittel 28, 38 in eine Kammer 27, 37, beispielsweise eine Spülkammer, der Blutbehandlungsvorrichtung eingesteckt. Ein Teil der Flüssigkeit aus dem Zufuhrsystem, welches über den Dialysewasseranschluss 1 zufließt, kann bei dieser Alternative dem Beutel mit Trockenkonzentrat, über ein Regelventil gesteuert, zugeführt werden.

Nach der Flüssigkeitszufuhr in den Trockenkonzentratbeutel wird die nun flüssige Konzentratlösung über die Kammer 27, 37, in welcher das jeweilige Verbindungsmittel 28, 38 steckt, analog dem Permeat zugeführt. Die Beutel mit Trockenkonzentrat werden hierfür an der Blutbehandlungsvorrichtung an entsprechenden Schnittstellen, beispielsweise an mit Zufuhr- und Abfuhrleitungen ausgebildeten Vorsprüngen, befestigt.

Als weitere Alternative kann die Konzentratversorgung des jeweiligen Konzentrats zentral erfolgen. Hierbei ist nicht an jeder Blutbehandlungsvorrichtung ein Konzentratbehälter vorgesehen. Vielmehr erfolgt die Zufuhr über einen Sammelkanister, welcher mehrere Blutbehandlungsvorrichtung mit Konzentrat versorgen kann. Die einzelnen Blutbehandlungsvorrichtungen haben hierfür einen Leitungsanschluss zu diesem Sammelkanister. Auch in diesem Fall der Konzentratversorgung ist das jeweilige Verbindungsmittel 28, 38 in die Kammer 27, 37 der Blutbehandlungsvorrichtung eingesteckt.

In dem Fall, in welchem die Konzentratversorgung über Trockenkonzentrat in Beuteln geschieht, wird detektiert, dass die Konzentratlösung nicht aus den Konzentratbehältern zugeführt wird. Erkannt wird diese Art der Konzentratversorgung beispielsweise durch zusätzliche Sensorik. Hierbei können Kontaktsensoren, etwa Hall-Sensoren, an einem Gehäusedeckel der Blutbehandlungsvorrichtung angeordnet sein, welcher mit der Befestigung eines Trockenkonzentratbeutels im Zusammenhang steht. Alternativ kann die Art der Konzentratversorgung manuell an der Blutbehandlungsvorrichtung eingestellt werden. Das Verbindungsmittel 28, 38 bleibt während dieser Art der Konzentratversorgung in der Kammer 27, 37 eingesteckt.

Auch in dem Fall, in welchem die Konzentratversorgung zentral erfolgt, kann dies über Sensorik erkannt werden. Hierfür kann an der entsprechenden Verbindungsstelle für die Anschlussleitung der Blutbehandlungsvorrichtung zu der zentralen Konzentratversorgung ein Kontaktsensor vorgesehen sein. Alternativ kann die Art der Konzentratversorgung manuell an der Blutbehandlungsvorrichtung ausgewählt werden.

Unabhängig von der Art der Konzentratversorgung erfolgt der Konzentratfluss über das entsprechende Verbindungsmittel 28, 38 und die entsprechende Konzentratzufuhrleitung 26, 36 zu dem Permeat, wobei der Konzentratfluss in den Konzentratzufuhrleitungen 26, 36 angeordnete Fluidsensoren, genauer den ersten Konzentratsensor 23 und zweiten Konzentratsensor 33 passiert. Wie zuvor beschrieben, sind diese in den Konzentratzufuhrleitungen 26, 36 ausgebildeten Konzentratsensoren 23, 33, Sensoren, welche Informationen über die in den Konzentratzufuhrleitungen 26, 36 vorherrschende Konzentratlösung liefern.

Sind diese in den Konzentratzufuhrleitungen 26, 36 angeordneten Konzentratsensoren 23, 33, wie in Fig. 2 dargestellt, als Leitwertsensoren ausgebildet, erkennen diese, ob eine Flüssigkeit in der Konzentratzufuhrleitung 26, 36 vorhanden ist. Diese Leitwertsensoren können zwischen einem Zustand - Leitfähigkeit erkannt - und einem Zustand - keine Leitfähigkeit erkannt - unterscheiden und damit auf das Vorhandensein eines Konzentratflusses Rückschluss geben. Geben die Konzentratsensoren 23, 33 ein Signal aus, dass keine Leitfähigkeit detektiert wurde, wird ein Beginn des Druckhaltetests verhindert.

Da die Blutbehandlungsvorrichtung während des Druckhaltetests bereits im Bypass-Betrieb ist, kann bei einer Erfassung einer unphysiologischen Flüssigkeit, beispielsweise einer Detektion von Luft in einer Konzentratzufuhrleitung 26, 36 (Zustand keine Leitfähigkeit erkannt), kein Umschalten mehr erfolgen. Anspruchsgemäß wird dieses Signal aber ausgewertet, um einen bereits gestarteten Druckhaltetest abzubrechen. Damit wird verhindert, dass Luft in den Dialysierflüssigkeitskreislauf gelangt. Kann in diesem frühen Zeitpunkt bereits eine unphysiologische Flüssigkeit erkannt werden, kann die Unterbrechung der Dialysebehandlung geringgehalten werden. Es wird kein Druckhaltetest durchgeführt, wenn feststeht, dass die Bedingung für einen Fluidwert nicht erfüllt ist. Die Bedingung für den Fluidwert ist nicht erfüllt, wenn eine unphysiologische Flüssigkeit vorliegt.

Wie zuvor beschrieben, wird durch Auswerten der Konzentratsensoren 23, 33 auch im Rahmen des Druckhaltetests nicht nur ein bereits gestarteter Druckhaltetest abgebrochen, auch wird der Beginn eines Druckhaltetest verhindert. Um Unterbrechungen der Dialysebehandlung gering zu halten, erfolgt damit anspruchsgemäß eine Auswertung der Konzentratsensoren 23, 33 auch im Rahmen des Druckhaltetests.

Zusätzlich zu den Konzentratsensoren 23, 33 können, wie oben beschrieben, Druckwertsensoren vorgesehen sein, welche die Art der Konzentratversorgung erkennen. Durch Auswerten dieser Sensoren ist es möglich, unphysiologische Flüssigkeit früh zu erkennen und ein Verhindern des Druckhaltetests zu bewirken. Erfolgt beispielsweise die Konzentratversorgung über mit Trockenkonzentrat gefüllten Beuteln, kann dies mittels Kontaktsensoren, etwa Hall-Sensoren, erkannt werden. Beispielsweise kann ein Kontaktsensor an einem Gehäusedeckel angeordnet sein, welcher zur Befestigung des Trockenkonzentratbeutels betätigt werden muss.

Ergibt die Auswertung des Signals eines Kontaktsensors, dass der Gehäusedeckel geöffnet ist, kann auf eine Konzentratversorgung mit Trockenkonzentrat geschlossen werden. In ähnlicher Weise kann zur Erkennung der zentralen Konzentratversorgung ein Kontaktsensor an einer Schnittstelle zur Anbindung des zentralen Schlauchsystems ausgebildet sein. Alternativ oder zusätzlich kann die Art der Konzentratversorgung manuell an der Blutbehandlungsvorrichtung ausgewählt werden.

Liegt eine Konzentratversorgung über Konzentratbehälter vor, kann das jeweilige Verbindungsmittel 28, 38 hierzu in den entsprechenden Konzentratbehälter eingesteckt sein. Wird aber erfasst, dass sich das jeweilige Verbindungsmittel 28, 38 in der Kammer 27, 37, befindet, resultiert aus dieser Tatsache, dass keine physiologische Flüssigkeit angesaugt werden kann. Bei dieser weiteren Abbruchbedingung kann zuerst erfasst werden, welche Art der Konzentratversorgung vorliegt.

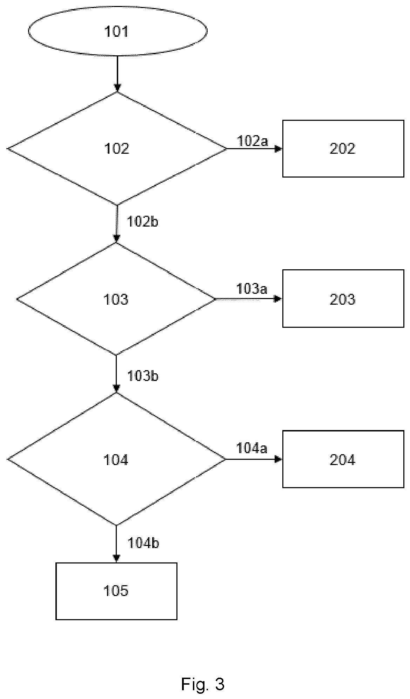

Anschließend wird die Position des entsprechenden Verbindungsmittels 28, 38 ermittelt, um zu erfassen, ob das Verbindungsmittel in den Konzentratbehälter oder in die Kammer 27, 37 der Blutbehandlungsvorrichtung eingesteckt ist, bzw. sich in dieser befindet. Dies kann beispielsweise über ein Auswerten von Kontaktsensoren erfolgen. Ein beispielhafter Ablauf dieser Prüfreihenfolge ist in Fig. 3 dargestellt.

Dabei zeigt der Schritt 101 den Beginn des Druckhaltetests an. Bei Schritt 102 erfolgt die Abfrage, ob der Fluidwert die vorgegebene Bedingung erfüllt. Erfüllt der Fluidwert die vorgegebene Bedingung (102b) wird in Schritt 103 ermittelt, ob der Konzentratversorgungsmodus ein erster Konzentratversorgungsmodus ist. Ist auch diese Bedingung erfüllt (103b), wird in Schritt 104 ermittelt, ob die Position des Verbindungsmittels 28, 38 die vorgegebene Bedingung erfüllt. Gibt die Ermittlung der entsprechenden Bedingungen ein positives Ergebnis (102b, 103b, 104b), so wird in Schritt 105 der Druckhaltetest durchgeführt.

Wird die jeweilige Bedingung nicht erfüllt, das heißt erfüllt der Fluidwert die vorgegebene Bedingung nicht, so wird in Schritt 202 der Druckhaltetest abgebrochen. Ebenso wird bei einem Nicht-Erfüllen der Bedingung, dass der Konzentratversorgungsmodus ein erster Konzentratversorgungsmodus ist, der Druckhaltetest in Schritt 203 abgebrochen. Auch bei einem Nicht-Erfüllen der Bedingung, dass die Position des Verbindungsmittels 28, 38 einer vorgegebenen Bedingung erfüllt, wird in Schritt 204 der Druckhaltetest abgebrochen.

Dabei kann die Reihenfolge der Schritte geändert werden, so dass zuerst die Position des Verbindungsmittels 28, 38 und anschließend die Art der Konzentratversorgung erfasst wird.

Zudem können zu den in Fig. 3 dargestellten Bedingungen weitere Bedingungen erfasst werden, um eine Entscheidung zur Durchführung des Druckhaltetests zu treffen. Ebenso müssen nicht alle in Fig. 3 dargestellten Bedingungen abgefragt werden. So kann es beispielsweise ausreichend sein, nur ein Vorliegen der Bedingung für den Fluidwert zu überprüfen.

Wird somit erkannt, dass das entsprechende Verbindungsmittel 28, 38 in die Kammer 27, 37 der Blutbehandlungsvorrichtung und nicht in den Konzentratbehälter eingesteckt ist, kann daraus rückgeschlossen werden, dass das Verbindungsmittel 28, 38 unphysiologische Flüssigkeit ansaugt. Um auch in diesem Fall zu verhindern, dass unphysiologische Flüssigkeit angesaugt wird und in den Dialysierflüssigkeitskreislauf gelangt, wird der Druckhaltetest verhindert, bzw. abgebrochen.

Wird hingegen erfasst, dass eine zentrale Konzentratversorgung oder eine Trockenkonzentratversorgung vorliegt, mit anderen Worten eine Konzentratversorgung bei welcher das Verbindungsmittel 28, 38 in die Kammer 27, 37 der Blutbehandlungsvorrichtung eingesteckt sein muss, erfolgt gerade kein Abbruch des Druckhaltetests.

Um zu erkennen, ob das Verbindungsmittel 28, 38 in die Kammer 27, 37 der Blutbehandlungsvorrichtung eingesteckt ist, können, wie zu Fig. 2 beschrieben ist, den jeweiligen Kammern 27, 37, in welche die Verbindungsmittel 28, 38 eingesteckt werden, jeweils Verbindungsmittelsensoren 29, 39 beziehungsweise Lageerkennungssensoren, zugeordnet sein. Hierfür können beispielsweise Magnetsensoren oder Kontaktsensoren, etwa Hall-Sensoren, verwendet werden. Alternativ können mechanische Schalter, etwa Druckschalter, Kipp- oder Wippschalter verwendet werden.

Durch Auswerten dieser zusätzlichen Information, der Art der Konzentratversorgung beispielsweise erhalten durch Verbindungsmittelsensoren 29, 39 sowie Fluidsensoren 13, 23, 33, insbesondere Konzentratsensoren in den Konzentratzufuhrleitungen 26, 36 im Rahmen des Druckhaltetests können unphysiologische Flüssigkeiten in einem frühen Zeitpunkt, genauer vor dem Erreichen des Dialysierflüssigkeitskreislaufs erkannt werden. Damit wird der Druckhaltetest in dem Fall nicht durchgeführt, wenn die vorgegebenen Bedingungen nicht erfüllt sind. Die vorgegebenen Bedingungen sind nicht erfüllt, wenn eine unphysiologische Flüssigkeit vorliegt.

Im Ergebnis werden Unterbrechungen der Dialysebehandlung geringgehalten, indem kein geschlossenes System ausgebildet wird, wenn die notwendigen Bedingungen nicht erfüllt sind. Zudem kann bei entsprechender Steuerung beispielsweise ein zeitintensives Spülen verhindert werden, um den Dialysierflüssigkeitskreislauf zu reinigen. Zudem wird durch die Verhinderung des Druckhaltetest bei Vorliegen einer unphysiologischen Flüssigkeit die Patientensicherheit erhöht.

Wird der Druckhaltetest durchgeführt, werden in dem Dialysierflüssigkeitskreislauf im Vergleich zu während einer Behandlung vorliegenden Drücken höhere Drücke hervorgerufen. Liegt unphysiologische Flüssigkeit in dem Dialysierflüssigkeitskreislauf vor, kann durch die anspruchsgemäße Lösung in jedem Fall verhindert werden, dass der Druckhaltetest durchgeführt und hohe Drücke erzeugt werden.

Die Steuerung kann einen Prozessor, beziehungsweise einen Mikrochip aufweisen, der in Verbindung mit einer Speichereinrichtung, in der ein Programmcode hinterlegt ist, konfiguriert, beziehungsweise programmiert sein kann, die entsprechenden Ansteuerungen in der Blutbehandlungsvorrichtung vorzunehmen. Der Prozessor beziehungsweise, der Mikrochip ist dazu eingerichtet, Daten zu verarbeiten und/oder unter anderem die Kommunikation zu übernehmen. Der Prozessor, beziehungsweise der Mikrochip kann durch Konfigurationsvorgaben programmiert werden und dient dann unter anderem auch als Verarbeitungseinheit zum Verarbeiten von Betriebs- und Maschinendaten.

## Patentansprüche

1. Blutbehandlungsvorrichtung, aufweisend
ein Fluidleitungssystem (10) zum Leiten eines Fluidflusses mit einem Leitungsabschnitt (14), wobei der Leitungsabschnitt (14) als ein geschlossenes Fluidsystem ausbildbar ist, und zumindest einer ersten Konzentratzufuhrleitung (26) zum Zuführen einer ersten Konzentratlösung;
einen Leitungszweig (15), welcher parallel zu zumindest einem Teilabschnitt des Leitungsabschnitts (14) ausgebildet ist, so dass bei einem Fluidfluss über den Leitungsabschnitt (14) ein Fluidfluss über den Leitungszweig (15) verhinderbar ist; und
eine Fluidpumpe (11) zum Fördern eines Fluids in dem Fluidleitungssystem (10);
ein Bestimmungsmittel (13; 23; 33; 29) zum Erfassen eines Zustands des Fluidleitungssystems (10);
eine Steuereinheit zum Steuern des Fluidflusses;
**dadurch gekennzeichnet, dass** die Steuereinheit derart konfiguriert ist, dass der Leitungsabschnitt (14) nur dann ein geschlossenes Fluidsystem zur Durchführung eines Druckhaltetests ausbildet, wenn der von dem Bestimmungsmittel (13; 23; 33; 29) erfasste Zustand eine vorgegebene Bedingung erfüllt.

2. Blutbehandlungsvorrichtung nach Anspruch 1, weiter aufweisend
einen ersten Konzentratsensor (23) zum Erfassen eines in der ersten Konzentratzufuhrleitung (26) vorherrschenden ersten Konzentratwerts, wobei das Bestimmungsmittel den erste Konzentratsensor (23) umfasst, und die vorgegebene Bedingung nur dann erfüllt ist, wenn der erste Konzentratwert eine erste Konzentratwertbedingung erfüllt; und/oder
einen Mischfluidsensor (13) zum Erfassen eines in dem Fluidleitungssystem (10) stromab der ersten Konzentratzufuhrleitung (26) vorherrschenden Mischfluidwerts, wobei das Bestimmungsmittel den Mischfluidsensor (13) umfasst, und die vorgegebene Bedingung nur dann erfüllt ist, wenn der Mischfluidwert eine Mischfluidwertbedingung erfüllt.

3. Blutbehandlungsvorrichtung nach Anspruch 1 oder 2, weiter aufweisend
ein Verbindungsmittel (28) zum Überführen der ersten Konzentratlösung in die erste Konzentratzufuhrleitung (26);
eine Kammer (27), in welche das Verbindungsmittel (28) einbringbar ist;
einen Verbindungsmittelsensor (29) zum Erfassen einer Position des Verbindungsmittels (28); und wobei
das Bestimmungsmittel den Verbindungsmittelsensor (29) umfasst, und die vorgegebene Bedingung nur dann erfüllt ist, wenn die Position des Verbindungsmittels (28) eine Positionsbedingung erfüllt.

4. Blutbehandlungsvorrichtung nach Anspruch 3, weiter aufweisend ein Mittel zum Erfassen eines Konzentratversorgungsmodus, wobei die vorgegebene Bedingung nur dann erfüllt ist, wenn eine Positionsbedingung erfüllt ist, und wenn der erfasste Konzentratversorgungsmodus ein Konzentratversorgungsmodus ist, in dem über das Verbindungsmittel (28) in der erfassten Position die Konzentratzufuhr erfolgt.

5. Blutbehandlungsvorrichtung nach einem der Ansprüche 1 bis 4, weiter aufweisend
eine zweite Konzentratzufuhrleitung (36) zum Zuführen einer zweiten Konzentratlösung, und
einen zweiten Konzentratsensor (33) zum Erfassen eines zweiten Konzentratwerts in der zweiten Konzentratzufuhrleitung (36), wobei das Bestimmungsmittel den zweiten Konzentratsensor (33) umfasst, und
die vorgegebene Bedingung nur dann erfüllt ist, wenn der zweite Konzentratwert eine zweite Konzentratwertbedingung erfüllt.

6. Blutbehandlungsvorrichtung nach einem der Ansprüche 3 bis 5, wobei
der Verbindungsmittelsensor einen Magnetsensor oder einen Kontaktsensor, insbesondere einen Hall-Sensor, oder einen mechanischen Schalter aufweist.

7. Blutbehandlungsvorrichtung nach einem der Ansprüche 3 bis 6, wobei
der Verbindungsmittelsensor (29) an der Kammer (27) der Blutbehandlungsvorrichtung angeordnet ist und/oder der Verbindungsmittelsensor (29) derart ausgebildet ist, dass er an einem der Blutbehandlungsvorrichtung zustellbarem Konzentratbehälter anbringbar ist.

8. Blutbehandlungsvorrichtung nach einem der Ansprüche 1 bis 7, wobei
der erste und/oder zweite Konzentratsensor (33, 23) ein Leitwertsensor oder ein Leitfähigkeitssensor oder ein Ultraschallsensor ist und/oder der Mischfluidsensor (13) ein Leitwertsensor oder ein Leitfähigkeitssensor oder ein Ultraschallsensor ist.

9. Blutbehandlungsvorrichtung nach einem der Ansprüche 1 bis 8, weiter aufweisend
einen Dialysator (9) in dem Leitungszweig (15).

10. Blutbehandlungsvorrichtung nach einem der Ansprüche 1 bis 9, weiter aufweisend
Absperrelemente (91, 92) in dem Fluidleitungssystem (10), wobei
die Steuereinheit derart konfiguriert ist, durch Ansteuern zumindest der Absperrelemente (91, 92) den Leitungsabschnitt (14) als ein geschlossenes Fluidsystem auszubilden.

11. Blutbehandlungsvorrichtung nach einem der Ansprüche 1 bis 10, weiter aufweisend
einen Drucksensor (16) zum Erfassen eines Druckwerts in dem Leitungsabschnitt (14), wobei
die Steuereinheit derart konfiguriert ist nur dann, wenn der von dem Bestimmungsmittel (13; 23; 33; 29) erfasste Zustand die vorgegebene Bedingung erfüllt,
in dem Leitungsabschnitt (14) einen Druckhaltetest durchzuführen, bei welchem Fluid in dem Leitungsabschnitt (14) mit Druck, bevorzugt von 600 bis 800 mmHg, noch bevorzugter von 700 bis 750 mmHg, beaufschlagt wird, und während eines vorgegebenen Zeitraums die Druckwerte in dem Leitungsabschnitt (14) erfasst werden, und aus einer Änderung der Druckwerte auf eine Leckage in dem Leitungsabschnitt (14) geschlossen werden kann.

12. Blutbehandlungsvorrichtung nach einem der Ansprüche 1 bis 11, weiter aufweisend
zumindest eine erste Konzentratpumpe (25) zum Fördern der ersten Konzentratlösung.

13. Verfahren zum Steuern eines Fluidflusses in einer Blutbehandlungsvorrichtung, wobei die Blutbehandlungsvorrichtung ein Fluidleitungssystem (10) zum Leiten eines Fluidflusses mit einem Leitungsabschnitt (14), der als ein geschlossenes Fluidsystem ausbildbar ist, und zumindest einer ersten Konzentratzufuhrleitung (26) zum Zuführen einer ersten Konzentratlösung, aufweist;
ferner die Behandlungsvorrichtung einen Leitungszweig (15) aufweist, welcher parallel zu zumindest einem Teilabschnitt des Leitungsabschnitts (14) ausgebildet ist, so dass bei einem Fluidfluss über den Leitungsabschnitt (14) ein Fluidfluss über den Leitungszweig (15) verhinderbar ist; aufweisend
Detektieren eines Zustands des Fluidleitungssystems mit einem Bestimmungsmittel (13; 23; 33; 29); und
Abgleichen des Zustands mit einer vorgegebenen Bedingung; **gekennzeichnet durch**
Zulassen, dass der Leitungsabschnitt (14) nur dann ein geschlossenes Fluidsystem zur Durchführung eines Druckhaltetests ausbildet, wenn der Zustand des Fluidleitungssystems (10) die vorgegebene Bedingung erfüllt.

14. Verfahren nach Anspruch 13, wobei die Blutbehandlungsvorrichtung einen ersten Konzentratsensor (23) als Bestimmungsmittel zum Erfassen eines in der ersten Konzentratzufuhrleitung (26) vorherrschenden ersten Konzentratwerts aufweist, und wobei die vorgegebene Bedingung nur dann erfüllt ist, wenn der Konzentratwert eine Konzentratwertbedingung erfüllt und/oder die Blutbehandlungsvorrichtung einen Mischfluidsensor (13) als Bestimmungsmittel zum Erfassen eines Mischfluidwerts in dem Fluidleitungssystem (10) stromabwärts der Konzentratzufuhrleitung (26) aufweist, und wobei die vorgegebene Bedingung nur dann erfüllt ist, wenn der Mischfluidwert eine Mischfluidwertbedingung erfüllt.

15. Verfahren nach Anspruch 13 oder 14, wobei die Blutbehandlungsvorrichtung eine Kammer (27) und ein in die Kammer (27) einbringbares Verbindungsmittel (28) sowie einen Verbindungsmittelsensor (29) als Bestimmungsmittel zum Erfassen einer Position des Verbindungsmittels (28) aufweist, weiter aufweisend
Detektieren einer Position des Verbindungsmittels (28) durch den Verbindungsmittelsensor (29), wobei
die vorgegebene Bedingung nur dann erfüllt ist, wenn die Position des Verbindungsmittels (28) eine Positionsbedingung erfüllt.

## Claims

1. A blood treatment device, comprising
a fluid line system (10) for guiding a fluid flow having a line portion (14), wherein the line portion (14) can be designed as a closed fluid system, and at least one first concentrate supply line (26) for supplying a first concentrate solution;
a line branch (15) which is formed parallel to at least one partial portion of the line portion (14), such that if fluid flows via the line portion (14), fluid flow via the line branch (15) can be prevented; and
a fluid pump (11) for conveying a fluid in the fluid line system (10);
a determining means (13; 23; 33; 29) for capturing a state of the fluid line system (10);
a control unit for controlling the fluid flow;
**characterized in that** the control unit is configured in such a way that the line portion (14) only forms a closed fluid system for carrying out a pressure holding test only when the state captured by the determining means (13; 23; 33; 29) meets a predetermined condition.

2. The blood treatment device according to claim 1, further comprising a first concentrate sensor (23) for capturing a first concentrate value prevailing in the first concentrate supply line (26), wherein the determining means comprises the first concentrate sensor (23), and the predetermined condition is only met if the first concentrate value meets a first concentrate value condition; and/or a mixed fluid sensor (13) for capturing a mixed fluid value prevailing in the fluid line system (10) downstream of the first concentrate supply line (26), wherein the determining means comprises the mixed fluid sensor (13), and the predetermined condition is only met if the mixed fluid value meets a mixed fluid value condition.

3. The blood treatment device according to claim 1 or 2, further comprising a connecting means (28) for transferring the first concentrate solution into the first concentrate supply line (26);
a chamber (27) into which the connecting means (28) can be inserted;
a connecting means sensor (29) for capturing a position of the connecting means (28); and wherein
the determining means comprises the connecting means sensor (29), and the predetermined condition is only met when the position of the connecting means (28) meets a position condition.

4. The blood treatment device according to claim 3, further comprising a means for capturing a concentrate provision mode, wherein
the predetermined condition is only met if a position condition is met, and if the captured concentrate provision mode is a concentrate provision mode in which the concentrate supply takes place via the connecting means (28) in the captured position.

5. The blood treatment device according to any one of claims 1 to 4, further comprising
a second concentrate supply line (36) for supplying a second concentrate solution, and
a second concentrate sensor (33) for capturing a second concentrate value in the second concentrate supply line (36), wherein the determining means comprises the second concentrate sensor (33), and
the predetermined condition is only met if the second concentrate value meets a second concentrate value condition.

6. The blood treatment device according to any one of claims 3 to 5, wherein
the connecting means sensor comprises a magnetic sensor or a contact sensor, in particular a Hall sensor, or a mechanical switch.

7. The blood treatment device according to any one of claims 3 to 6, wherein
the connecting means sensor (29) is arranged on the chamber (27) of the blood treatment device and/or the connecting medium sensor (29) is designed in such a way that it can be attached to a concentrate container which can be supplied to the blood treatment device.

8. The blood treatment device according to any one of claims 1 to 7, wherein
the first and/or second concentrate sensor (33, 23) is a conductance sensor or a conductivity sensor or an ultrasonic sensor and/or the mixed fluid sensor (13) is a conductance sensor or a conductivity sensor or an ultrasonic sensor.

9. The blood treatment device according to any one of claims 1 to 8, further comprising
a dialyzer (9) in the line branch (15).

10. The blood treatment device according to any one of claims 1 to 9, further comprising
shut-off elements (91, 92) in the fluid line system (10), wherein
the control unit is configured in such a way as to form the line portion (14) as a closed fluid system by controlling at least the shut-off elements (91, 92).

11. The blood treatment device according to any one of claims 1 to 10, further comprising
a pressure sensor (16) for capturing a pressure value in the line portion (14), wherein
the control unit is configured in such a way, only when the state captured by the determining means (13; 23; 33; 29) meets the predetermined condition,
to carry out a pressure holding test in the line portion (14), in which fluid in the line portion (14) is subjected to pressure, preferably 600 to 800 mmHg, more preferably 700 to 750 mmHg, and the pressure values in the line portion (14) are captured during a predetermined time period, and a leakage in the line portion (14) can be inferred from a change in the pressure values.

12. The blood treatment device according to any one of claims 1 to 11, further comprising
at least one first concentrate pump (25) for conveying the first concentrate solution.

13. A method for controlling a fluid flow in a blood treatment device, wherein the blood treatment device comprises a fluid line system (10) for guiding a fluid flow having a line portion (14) which can be designed as a closed fluid system, and at least one first concentrate supply line (26) for supplying a first concentrate solution;
the treatment device further comprises a line branch (15) which is formed parallel to at least one partial portion of the line portion (14), such that if fluid flows via the line portion (14), fluid flow via the line branch (15) can be prevented; comprising
detecting a state of the fluid line system using a determining means (13; 23; 33; 29); and
comparing the state with a predetermined condition; **characterized by** allowing the line portion (14) to form a closed fluid system for carrying out a pressure holding test only when the state of the fluid line system (10) meets the predetermined condition.

14. The method according to claim 13, wherein the blood treatment device comprises a first concentrate sensor (23) as a determining means for capturing a first concentrate value prevailing in the first concentrate supply line (26), and wherein the predetermined condition is only met if the concentrate value meets a concentrate value condition and/or the blood treatment device comprises a mixed fluid sensor (13) as a determining means for capturing a mixed fluid value in the fluid line system (10) downstream of the concentrate supply line (26), and wherein the predetermined condition is only met if the mixed fluid value meets a mixed fluid value condition.

15. The method according to claim 13 or 14, wherein the blood treatment device comprises a chamber (27) and a connecting means (28) which can be inserted into the chamber (27) as well as a connecting means sensor (29) as a determining means for capturing a position of the connecting means (28), further comprising detecting the position of the connecting means (28) by means of the connecting means sensor (29), wherein
the predetermined condition is only met if the position of the connecting means (28) meets a position condition.

## Revendications

1. Dispositif de traitement du sang, comprenant
un système de conduite de fluide (10) pour conduire un écoulement de fluide à l'aide d'une section de conduite (14), la section de conduite (14) pouvant être conçue sous la forme d'un système de fluide fermé, et au moins une première conduite d'alimentation en concentré (26) pour fournir une première solution de concentré ;
une branche de conduite (15), laquelle est conçue parallèlement à au moins une sous-section de la section de conduite (14) de telle sorte que, lors d'un écoulement de fluide par l'intermédiaire de la section de conduite (14), il est possible d'empêcher un écoulement de fluide par l'intermédiaire de la branche de conduite (15) ; et
une pompe à fluide (11) pour transporter un fluide dans le système de conduite de fluide (10) ;
un moyen de détermination (13 ; 23 ; 33 ; 29) pour détecter un état du système de conduite de fluide (10) ;
une unité de commande pour commander l'écoulement de fluide ;
**caractérisé en ce que** l'unité de commande est conçue de telle sorte que la section de conduite (14) forme un système de fluide fermé pour mettre en œuvre un essai de maintien de pression seulement si l'état détecté à l'aide du moyen de détermination (13 ; 23 ; 33 ; 29) répond à une condition prédéterminée.

2. Dispositif de traitement du sang selon la revendication 1, comprenant en outre un premier capteur de concentré (23) pour détecter une première valeur de concentré prédominante dans la première conduite d'alimentation en concentré (26), dans lequel le moyen de détermination comporte le premier capteur de concentré (23), et la condition prédéterminée est remplie seulement si la première valeur de concentré satisfait à la première condition de la valeur de concentré ; et/ou
un capteur de fluide mixte (13) pour détecter une valeur de fluide mixte prédominante dans le système de conduite de fluide (10) en aval de la première conduite d'alimentation en concentré (26), dans lequel le moyen de détermination comprend le capteur de fluide mixte (13), et la condition prédéterminée est remplie seulement si la valeur de fluide mixte remplit une condition de la valeur de fluide mixte.

3. Dispositif de traitement du sang selon la revendication 1 ou 2, comprenant en outre
un moyen de connexion (28) pour transférer la première solution de concentré dans la première conduite d'alimentation en concentré (26) ;
une chambre (27) dans laquelle le moyen de connexion (28) peut être inséré ;
un capteur de moyen de connexion (29) pour détecter une position du moyen de connexion (28) ; et dans lequel
le moyen de détermination comprend le capteur de moyen de connexion (29), et la condition prédéterminée n'est satisfaite que si la position du moyen de connexion (28) satisfait à une condition de position.

4. Dispositif de traitement du sang selon la revendication 3, comprenant en outre un moyen destiné à détecter un mode d'alimentation en concentré, dans lequel la condition prédéterminée est remplie seulement si une condition de position est remplie, et si le mode d'alimentation en concentré détecté est un mode d'alimentation en concentré dans lequel l'alimentation en concentré est effectuée par l'intermédiaire du moyen de connexion (28) dans la position détectée.

5. Dispositif de traitement du sang selon l'une quelconque des revendications 1 à 4, comprenant en outre
une seconde conduite d'alimentation en concentré (36) pour acheminer une seconde solution de concentré, et
un second capteur de concentré (33) pour détecter une seconde valeur de concentré dans la seconde conduite d'alimentation en concentré (36), dans lequel le moyen de détermination comprend le second capteur de concentré (33), et
la condition prédéterminée est remplie seulement si la seconde valeur de concentré satisfait à une seconde condition de la valeur de concentré.

6. Dispositif de traitement du sang selon l'une quelconque des revendications 3 à 5, dans lequel
le capteur de moyen de connexion comprend un capteur magnétique ou un capteur de contact, en particulier un capteur à effet Hall, ou un interrupteur mécanique.

7. Dispositif de traitement du sang selon l'une quelconque des revendications 3 à 6, dans lequel
le capteur de moyen de connexion (29) est disposé sur la chambre (27) du dispositif de traitement du sang et/ou le capteur de moyen de connexion (29) est conçu de telle sorte qu'il peut être fixé sur un récipient de concentré pouvant être fourni au dispositif de traitement du sang.

8. Dispositif de traitement du sang selon l'une quelconque des revendications 1 à 7, dans lequel
le premier et/ou le second capteur de concentré (33, 23) sont un capteur de conductance ou un capteur de conductivité ou un capteur à ultrasons et/ou le capteur de fluide mixte (13) est un capteur de conductance ou un capteur de conductivité ou un capteur à ultrasons.

9. Dispositif de traitement du sang selon l'une quelconque des revendications 1 à 8, comprenant en outre
un dialyseur (9) dans la branche de conduite (15).

10. Dispositif de traitement du sang selon l'une quelconque des revendications 1 à 9, comprenant en outre
des éléments d'arrêt (91, 92) dans le système de conduite de fluide (10), dans lequel
l'unité de commande est conçue de telle sorte qu'au moyen de la commande des éléments d'arrêt (91, 92), la section de conduite (14) forme un système de fluide fermé.

11. Dispositif de traitement du sang selon l'une quelconque des revendications 1 à 10, comprenant en outre
un capteur de pression (16) pour détecter une valeur de pression dans la section de conduite (14), dans lequel
l'unité de commande est conçue, seulement si l'état détecté à l'aide du moyen de détermination (13 ; 23 ; 33 ; 29) satisfait à la condition prédéterminée,
pour mettre en œuvre un essai de maintien de pression dans la section de conduite (14) lors duquel le fluide dans la section de conduite (14) est soumis à une pression, de préférence dans la plage comprise entre 600 et 800 mmHg, plus particulièrement entre 700 et 750 mmHg, et les valeurs de pression dans la section de conduite (14) sont déterminées pendant une période prédéterminée, et un changement des valeurs de pression peut permettre de conclure à une fuite dans la section de conduite (14).

12. Dispositif de traitement du sang selon l'une quelconque des revendications 1 à 11, comprenant en outre
au moins une première pompe à concentré (25) pour transporter la première solution de concentré.

13. Procédé de commande d'un écoulement de fluide dans un dispositif de traitement du sang, dans lequel le dispositif de traitement du sang comprend un système de conduite de fluide (10) pour conduire un écoulement de fluide à l'aide d'une section de conduite (14), lequel peut être conçu sous la forme d'un système de fluide fermé, et comprend au moins une première conduite d'alimentation en concentré (26) pour acheminer une première solution de concentré ;
le dispositif de traitement comporte en outre une branche de conduite (15) conçue parallèlement par rapport à au moins une section partielle de la section de conduite (14) de telle sorte que, lors d'un écoulement de fluide par l'intermédiaire de la section de conduite (14), il est possible d'empêcher un écoulement de fluide par l'intermédiaire de la branche de conduite (15) ; comprenant
la détection d'un état du système de conduite de fluide à l'aide d'un moyen de détermination (13 ; 23 ; 33 ; 29) ; et
la comparaison de l'état avec une condition prédéterminée ; **caractérisé par** la permission que la section de conduite (14) forme un système de fluide fermé pour mettre en œuvre un essai de maintien de pression seulement si l'état du système de conduite de fluide (10) répond à la condition prédéterminée.

14. Procédé selon la revendication 13, dans lequel le dispositif de traitement du sang comporte un premier capteur de concentré (23) servant de moyen de détermination pour détecter une première valeur de concentré prédominante dans la première conduite d'alimentation en concentré (26), et dans lequel la condition prédéterminée est remplie seulement si la valeur de concentré satisfait à une condition de la valeur de concentré et/ou le dispositif de traitement du sang comporte un capteur de fluide mixte (13) servant de moyen de détermination pour détecter une valeur de fluide mixte dans le système de conduite de fluide (10) en aval de la conduite d'alimentation en concentré (26), et dans lequel la condition prédéterminée est remplie seulement si la valeur de fluide mixte satisfait à une condition de la valeur de fluide mixte.

15. Procédé selon la revendication 13 ou 14, dans lequel le dispositif de traitement du sang comprend une chambre (27) et un moyen de connexion (28) pouvant être inséré dans la chambre (27), ainsi qu'un capteur de moyen de connexion (29) servant de moyen de détermination d'une position du moyen de connexion (28), comprenant en outre
la détection d'une position du moyen de connexion (28) à l'aide du capteur de moyen de connexion (29), dans lequel
la condition prédéterminée est satisfaite seulement si la position du moyen de connexion (28) satisfait à la condition de position.
